(19) Europäisches Patentamt

European Patent Office

Office européen des brevets

(11) **EP 0 913 152 B1**

(12) **EUROPÄISCHE PATENTSCHRIFT**

(45) Veröffentlichungstag und Bekanntmachung des Hinweises auf die Patenterteilung:
**19.12.2001 Patentblatt 2001/51**

(51) Int Cl.[7]: **A61K 31/485**, A61K 47/18

(21) Anmeldenummer: **98120639.4**

(22) Anmeldetag: **03.11.1998**

(54) **Stabilisiertes Kombinationsarzneimittel enthaltend Naloxone und ein Opiatanalgetikum**

Stabilised combination of drugs comprising naloxone and an opioid analgesic

Composition stabilisée d'une combinaison de substances actives comprenant de la naloxone et un opioide analgésique

(84) Benannte Vertragsstaaten:
AT BE CH DE DK ES FR GB GR IT LI NL PT

(30) Priorität: 03.11.1997 DE 29719483 U
18.11.1997 DE 29720448 U
23.07.1998 DE 19833028

(43) Veröffentlichungstag der Anmeldung:
**06.05.1999 Patentblatt 1999/18**

(73) Patentinhaber: **STADA ARZNEIMITTEL AG**
**61118 Bad Vilbel (DE)**

(72) Erfinder:
• **Hansen, Peter, Dr.**
**63303 Dreieich (DE)**
• **Hofmann, Herbert**
**61206 Wöllstadt (DE)**
• **Schumann, Christof**
**35767 Breitscheid-Erdbach (DE)**

(74) Vertreter: **Hamm, Volker, Dr.**
**Maiwald Patentanwalts-GmbH Postfach 11 34 17**
**20434 Hamburg (DE)**

(56) Entgegenhaltungen:
EP-A- 0 144 243     WO-A-94/10129
WO-A-97/04835     WO-A-98/35679
DE-A- 4 423 850

• DATABASE WPI Section Ch, Week 9604 Derwent Publications Ltd., London, GB; Class A96, AN 96-035828 XP002092829 -& JP 07 304673 A (SEKISUI CHEM CO LTD) , 21. November 1995 -& CHEMICAL ABSTRACTS, vol. 124, no. 10, 4. März 1996 Columbus, Ohio, US; abstract no. 121173, COTA T. ET AL: "Transdermal adhesive preparations containing morphine and its anatgonists" XP002092828 -& PATENT ABSTRACTS OF JAPAN vol. 096, no. 003, 29. März 1996 & JP 07 304673 A (SEKISUI CHEM CO LTD), 21. November 1995

• FEY H. ET AL: "Wörterbuch der Kosmetik" 1990 , WVG , STUTTGART XP002092827 169750 * Seite 243 * * Seite 50 *

**Beschreibung**

Die vorliegende Erfindung betrifft pharmazeutische Zusammensetzungen, welche Naloxon oder ein pharmazeutisch verträgliches Salz davon und Ethylendiamintetraessigsäure oder ein pharmazeutisch verträgliches Salz davon enthalten.

Im Stand der Technik sind Arzneimittel bekannt, die Naloxon in Kombination mit beispielsweise Tilidin enthalten. Tilidin ist ein potentes Schmerzmittel zur Behandlung von starken oder sehr starken Schmerzen und gehört zur Gruppe der morphinartig wirkenden Analgetika. Das in solchen Kombinationspräparaten enthalten Naloxon ist ein Morphin-Antagonist und soll die mißbräuchliche Anwendung des Opiatanalgetikums, mit dem eine starke Suchtgefahr verbunden ist, in Kombinationsfertigarzneimitteln verhindern.

Naloxon hat eine dem Morphin ähnliche Grundstruktur und kann wie dieses über einen radikalischen Prozeß zu einem Dimeren, dem 2,2'-Bisnaloxon bzw. Pseudonaloxon, reagieren. Diese Reaktion kann von Metallionen, beispielsweise Eisenionen, katalysiert werden. Durch diese katalytische Reaktion des Naloxons wird die Wirkung als Morphin-Antagonist vermindert, was zu einem nachteiligen bzw. mißbräuchlichen Einsatz des Fertigarzneimittels führen kann.

In dem erst nach dem Prioritätstag der vorliegenden Anmeldung veröffentlichten deutschen Gebrauchsmuster 297 19 704 wird die Stabilisierung Naloxon-haltiger Zusammensetzungen durch Zusatz bestimmter, die Bildung des 2,2'-Bisnaloxons zurückdrängender Verbindungen beschrieben. Als geeignete Stabilisatoren werden Tocopherolacetat, Ascorbinsäure und als bevorzugte Substanz Natriumbisulfit exemplifiziert, die den zu stabilisierenden Zusammensetzungen in einer Menge von mindestens 0,001 Gew.-% zugesetzt werden müssen. Ein Hinweis auf stabilisierte Kombinationspräparate umfassend Naloxon und ein Opiatanalgetikum wie beispielsweise Tilidin findet sich in diesem Gebrauchsmuster nicht Auch findet sich kein Hinweis darauf, daß der Stabilisator in aus pharmazeutischer Sicht vorteilhaften niedrigen Gehalten zugesetzt werden könnte.

Der vorliegenden Erfindung liegt die Aufgabe zugrunde, eine pharmazeutische Zusammensetzung bereitzustellen, welche mindestens ein Opiatanalgetikum und Naloxon in pharmazeutisch wirksamen Mengen enthält, wobei diese pharmazeutische Zusammensetzung lagerstabil sein soll, d.h. insbesondere die Dimerisierung von Naloxon verhindert werden soll.

Diese Aufgabe wird durch die im unabhängigen Anspruch definierte Zusammensetzung gelöst.

Die abhängigen Ansprüche definieren vorteilhafte Ausführungsformen der Erfindung.

Erfindungsgemäß wird eine pharmazeutische Zusammensetzung angegeben, die mindestens ein Opiatanalgetikum oder ein pharmazeutisch verträgliches Salz davon, Naloxon oder ein pharmazeutisch verträgliches Salz davon, wie Naloxon $\cdot$HCl$\cdot$2H$_2$O, und Ethylendiamintetraessigsäure oder ein pharmazeutisch verträgliches Salz davon enthält. Beispiele für Opiatanalgetika sind Tilidin, Tilidin$\cdot$HCl, Morphin, Hydromorphon, Oxycodon, Pethidin, Pentazosin und Buprenorphin.

In einer bevorzugten Ausführungsform der vorliegenden Erfindung wird ein Salz der Ethylendiamintetraessigsäure (EDTA) verwendet, das vorzugsweise in einer Konzentration von 0,000001 bis 0,1 Gew.-%, mehr bevorzugt 0,0005 bis 0,01 Gew.-%, am meisten bevorzugt 0,001 bis 0,002 Gew.-%, bezogen auf die Gesamtmenge der pharmazeutischen Zusammensetzung, vorliegt. Vorzugsweise wird das Natriumsalz der Ethylendiamintetraessigsäure verwendet

Die Konfektionierung der erfindungsgemäßen pharmazeutischen Zusammensetzung unterliegt keiner besonderen Beschränkung, und sie kann in flüssiger, gelartiger oder fester Darreichungsform vorliegen.

Es ist zwar bekannt, daß die die Dimerisierung des Naloxons möglicherweise katalysierenden Eisenionen durch Komplexbildner, wie beispielsweise Salze von EDTA, komplexiert werden können, jedoch scheinen bei der vorstehend genannten Dimerisierungsreaktion von Naloxon zu 2,2'-Bisnaloxon noch weitere Faktoren eine Rolle spielen. So zeigen auch in Glasgefäßen hergestellte Formulierungen, die keinerlei Metallkontakt hatten, in sogenannten Streßversuchen einen deutlichen Anstieg des Gehalts an 2,2'-Bisnaloxon. In den der vorliegenden Erfindung zugrunde liegenden Untersuchungen wurde ferner festgestellt, daß auch das in den erfindungsgemäßen Zusammensetzungen enthaltende Opiatanalgetikum die Dimerisierung des Naloxons fördert.

Der pH-Wert von z.B. Tilidin$\cdot$HCl und Naloxon$\cdot$HCl enthaltenden Zusammensetzungen liegt üblicherweise bei etwa 1,5. Bei diesem pH-Wert liegen die Salze von EDTA aber in der zur Metallionenkomplexierung unwirksamen Säureform vor.

Überraschenderweise wurde jedoch festgestellt, daß durch Zusatz von EDTA-Salzen zu Naloxon enthaltenden Opiatanalgetika-Formulierungen die Dimerisierung von Naloxon zu 2,2'-Bisnaloxon vollständig verhindert bzw. vermindert werden kann.

Die nachstehenden Beispiele erläutern die vorliegende Erfindung.

### Beispiel 1

Eine rein wäßrige Formulierung des Opiatanalgetikums Tilidin·HCl in Kombination mit Naloxon wird durch Zusatz von 0,0015 Gew.-% Natrium-EDTA stabilisiert. Die Formulierung hat folgende Zusammensetzung:

| 1,0 ml Lösung enthalten: | |
|---|---:|
| Tilidin·HCl·0,5 $H_2O$ | 71,458 mg |
| Naloxon·HCl·$H_2O$ | 6,080 mg |
| Natriumbenzoat | 1,20 mg |
| Salzsäure 25% | 4,75 mg |
| Natrium-EDTA (Dinatrium-Salz) | 0,015 mg |
| Gereinigtes Wasser | 927,9865 mg |
| | 1011,476 mg |

### Beispiel 2

Eine wäßrig/ethanolische Formulierung des Opiatanalgetikums Tilidin·HCl in Kombination mit Naloxon wird durch Zusatz von 0,00097 Gew.-% Na-EDTA stabilisiert. Die Formulierung hat folgende Zusammensetzung:

| 0,72 ml Lösung enthalten: | |
|---|---:|
| Tilidin·HCl·0,5 $H_2O$ | 51,45 mg |
| Naloxon·HCl·$H_2O$ | 4,40 mg |
| Ethanol 96% | 72,10 mg |
| Salzsäure 25% | 1,96 mg |
| Natrium-EDTA (Dinatrium-Salz) | 0,007 mg |
| Gereinigtes Wasser | 587,21 mg |
| | 717,127 mg |

### Beispiel 3

Formulierungen gemäß Beispiel 2 mit oder ohne Zusatz von Natrium-EDTA wurden einerseits in einem Metallgefäß und andererseits in einem Glasgefäß hergestellt und anschließend einem Streßtest (2 bzw. 5 Tage bei 60°) unterworfen. Die Ergebnisse sind in der nachstehenden Tabelle 1 dargestellt.

Tabelle 1

| | Ausgangswert | Streßtest 2 Tage 60°C | Streßtest 5 Tage 60°C |
|---|---|---|---|
| ohne Natrium-EDTA Zusatz (hergestellt in einem Metallgefäß) | 0,125% | 2,406% | 3,529% |
| mit Natrium-EDTA Zusatz (hergestellt in einem Metallgefäß) | 0,156% | 0,143% | 0,142% |
| ohne Natrium-EDTA Zusatz (hergestellt in einem Glasgefäß) | 0,176% | 2,696% | nicht geprüft |
| mit Natrium-EDTA Zusatz (hergestellt in einem Glasgefäß) | 0,102% | 0,124% | 0,119% |

Die Ergebnisse zeigen deutlich, daß der Zusatz von Natrium-EDTA zu einer Tilidin und Naloxon enthaltenden Formulierung eine Dimerisierung von Naloxon zu 2,2'-Bisnaloxon verhindert, und demgemäß die Lagerstabilität derartiger Arzneimittelformulierungen mit einem Zusatz an EDTA deutlich verbessert ist.

### Beispiel 4

Formulierungen gemäß Beispiel 2 mit variablen Gehalten an Natrium-EDTA werden Streßtests unterzogen und die Gehalte an 2,2'-Bisnaloxon bestimmt. Die erhaltenen Ergebnisse sind in Tabelle 2 zusammengefaßt.

Tabelle 2:

| Stabilisierender Einfluß von EDTA in Abhängigkeit von der EDTA-Konzentration | | | |
|---|---|---|---|
| Natriumedetat konz. | Gehalt an 2,2'-Anfangswert | Bisnaloxon gelagert bei 3 Tage | 60°C 2Wochen |
| 0,0000% | 0,084% | 3,8% | 5,14% |
| 0,001% (10 ppm) | 0,059% | 0,077% | 0,104% |
| 0,0005% (5 ppm) | 0,065% | 0,084% | 0,103% |
| 0,0002% (2 ppm) | 0,055% | 0,096% | 0,120% |
| 0,0001% (1 ppm) | 0,057% | 0,098% | 0,143% |
| 0,00005% (0,5ppm) | 0,057% | 0,109% | 0,162% |

Die tabellierten Werte zeigen deutlich, daß das Natrium-EDTA selbst bei einer Konzentration von 0,5 ppm noch einen stabilisierenden Einfluß ausübt.

Beispiel 5

a) Das in Beispiel 2 beschriebene Tilidin/Naloxon-Kombinationspräparat wird mit Natrium-EDTA-Gehalten von 0,002 und 0,0005 Gew.-% formuliert. Zu Vergleichszwecken wird eine identische Zusammensetzung präpariert, die jedoch statt Natrium-EDTA 0,0005 Gew.-% Natriumbisulfit als Stabilisator enthält.

Die Zusammensetzungen werden wiederum einem Streßtest unterzogen und die Gehalte an 2,2'-Bisnaloxon bestimmt. Die erhaltenen Ergebnisse sind in Tabelle 3 zusammengefaßt.

Tabelle 3:

| Stabilisierende Wirkung von Natriumbisulfit im Vergleich zu Natrium-EDTA bei Tilidin/Naloxon-Kombinationspräparaten | | | |
|---|---|---|---|
| | Gehalt an 2,2' | -Bisnaloxon | |
| | 0 Tage | 3 Tage 60°C | 14 Tage 60°C |
| Tilidin/Naloxon Kombination ohne Stabilisator | 0,124% | 6,564% | 6,829% |
| 0,002% Na-EDTA | 0,097% | 0,101% | 0,086% |
| 0,0005% Natriumbisulfit | 0,089% | 3,501% | 5,923% |
| 0,0005% Na-EDTA | 0,087% | 0,104% | 0,105% |

Es wird deutlich, daß die stabilisierende Wirkung von Natriumbisulfit bei geringeren Konzentrationen im Gegensatz zu der von EDTA kaum nachweisbar ist.

b) Es werden wie zuvor beschriebene Zusammensetzungen unter Zusatz von Natrium-EDTA bzw. Natriumbisulfit hergestellt, allerdings ohne Zusatz von Tilidin. Diese "Monopräparate" werden wiederum Streßtests unterzogen und die 2,2'-Bisnaloxongehalte bestimmt. Die hierbei erhaltenen Ergebnisse sind in Tabelle 4 zusammengefaßt.

Tabelle 4:

| Stabilisierende Wirkung von Natriumbisulfit im Vergleich zu Natrium-EDTA bei Naloxon-Monopräparaten | | | |
|---|---|---|---|
| | Gehalt an 2,2'- | Bisnaloxon | |
| | 0 Tage | 3 Tage 60°C | 14 Tage 60°C |
| Naloxon Mono Rezeptur ohne Stabilisator | 0,079% | 1,530% | 3,480% |
| 0,002% Na-EDTA | 0,077% | 0,077% | 0,077% |

Tabelle 4:   (fortgesetzt)

| Stabilisierende Wirkung von Natriumbisulfit im Vergleich zu Natrium-EDTA bei Naloxon-Monopräparaten | | | |
|---|---|---|---|
| | Gehalt an 2,2'- | Bisnaloxon | |
| | 0 Tage | 3 Tage 60°C | 14 Tage 60°C |
| 0,0005% Natriumbisulfit | 0,082% | 0,081% | 0,780% |
| 0,0005% Na-EDTA | 0,077% | 0,099% | 0,085% |

Es zeigt sich, daß die Mengen an gebildetem 2,2'-Bisnaloxon im Monopräparat sehr viel geringer sind, als bei dem Tilidin/Naloxon-Kombinationspräparat, daß also das Tilidin eine entscheidende Rolle für die Dimerisierung des Naloxons spielt. Auch bei den Monopräparaten ist das Natrium-EDTA dem Natriumbisulfit bei niedrigen Konzentrationen hinsichtlich der stabilisierenden Wirkung deutlich überlegen.

**Patentansprüche**

1. Pharmazeutische Zusammensetzung, enthaltend mindestens ein Opiatanalgetikum oder ein pharmazeutisch verträgliches Salz davon, Naloxon oder ein pharmazeutisch verträgliches Salz davon und Ethylendiamintetraessigsäure oder ein pharmazeutisch verträgliches Salz davon.

2. Pharmazeutische Zusammensetzung nach Anspruch 1, wobei das Opiatanalgetikum Tilidin und dessen pharmazeutisch verträgliches Salz Tilidinhydrochlorid ist.

3. Pharmazeutische Zusammensetzung nach Anspruch 1 oder 2, wobei die Ethylendiamintetraessigsäure bzw. deren Salz in einer Konzentration von 0,000001 bis 0,1 Gew.-%, bevorzugt 0,0005 bis 0,01 Gew.-%, mehr bevorzugt 0,001 bis 0,002 Gew.-%, bezogen auf die Gesamtmenge der pharmazeutischen Zusammensetzung, vorliegt.

4. Pharmazeutische Zusammensetzung nach einem der Ansprüche 1 bis 3, welche in flüssiger, gelartiger oder fester Darreichungsform vorliegt.

**Claims**

1. Pharmaceutical composition comprising at least one opiate analgesic or a pharmaceutically acceptable salt thereof, naloxone or a pharmaceutically acceptable salt thereof and ethylenediamine tetraacetic acid or a pharmaceutically acceptable salt thereof.

2. Pharmaceutical composition according to claim 1, whereby the opiate analgesic is tilidin and its pharmaceutically acceptable salt is tilidin hydrochloride.

3. Pharmaceutical composition according to claim 1 or 2, whereby ethylendiamine tetraacetic acid or its salt is present in a concentration of from 0.000001 to 0.1 wt.-%, preferably from 0.0005 to 0.01 wt.-%, more preferred from 0.001 to 0.002 wt.-%, based on the total weight of the pharmaceutical composition.

4. Pharmaceutical composition according to one of claims 1 to 3 in a liquid, gel-like or solid form.

**Revendications**

1. Composition pharmaceutique, contenant au moins un opioïde analgésique ou un sel de qualité pharmaceutique de celui-ci, de la naloxone ou un sel de qualité pharmaceutique de celle-ci et de l'acide tétraacétique d'éthylènediamine ou un sel pharmaceutique de celui-ci.

2. Composition pharmaceutique selon la revendication 1, **caractérisée en ce que** l'opioïde analgésique est la tilidine et son sel de qualité pharmaceutique, l'hydrochlorure de tilidine.

**3.** Composition pharmaceutique selon la revendication 1 ou 2, **caractérisée en ce que** la teneur de la composition pharmaceutique en acide tétraacétique d'éthylènediamine ou en son sel est comprise entre 0,000001 et 0,1 % en poids, de préférence entre 0,0005 et 0,01 % en poids, et de manière encore plus préférentielle entre 0,001 et 0,002 % en poids.

**4.** Composition pharmaceutique selon l'une des revendications 1 à 3, **caractérisé en ce qu'**elle existe sous les formes galéniques de liquide, gel ou solide.